# EUROPEAN PATENT SPECIFICATION

(11) **EP 2 848 691 B1**
(45) Date of publication and mention of the grant of the patent: **12.04.2017**
(21) Application number: 13183909.4
(22) Date of filing: 11.09.2013
(51) Int. Cl.: C12N 15/74, C12N 15/66, C12N 15/70, C12N 15/10

(54) **BROAD HOST RANGE EXPRESSION VECTOR FOR DIVERSE PROKARYOTES**
EXPRESSIONSVEKTOR MIT BREITEM WIRTSSPEKTRUM FÜR DIVERSE PROKARYOTEN
VECTEUR D'EXPRESSION À LARGE PLAGE D'HÔTES POUR DIVERS PROKARYOTES

(43) Date of publication of application: 18.03.2015
(73) Proprietor: Westfälische Wilhelms-Universität Münster, 48149 Münster (DE)
(72) Inventor: Kolkenbrock, Stephan, 40597 Düsseldorf (DE); Moerschbacher, Bruno, 48161 Münster (DE)
(74) Representative: Remus, Alvaro Johannes

(56) References cited:
- WO-A2-2007/038276
- US-A1- 2007 059 768
- BELEV T N ET AL: "A fully modular vector system for the optimization of gene expression in Escherichia coli", PLASMID, NEW YORK,NY, US, vol. 26, no. 2, 1 September 1991 (1991-09-01), pages 147-150, XP024799807, ISSN: 0147-619X, DOI: 10.1016/0147-619X(91)90056-3 [retrieved on 1991-09-01]
- JAMIE E. PRIOR ET AL: "Broad-host-range vectors for protein expression across gram negative hosts", BIOTECHNOLOGY AND BIOENGINEERING, 10 February 2010 (2010-02-10), pages n/a-n/a, XP055097226, ISSN: 0006-3592, DOI: 10.1002/bit.22695
- I PEREZARELLANO: "Construction of Compatible Wide-Host-Range Shuttle Vectors for Lactic Acid Bacteria and Escherichia coli", PLASMID, vol. 46, no. 2, 1 September 2001 (2001-09-01), pages 106-116, XP055097220, ISSN: 0147-619X, DOI: 10.1006/plas.2001.1531
- LEENHOUTS K J ET AL: "Nucleotide sequence and characterization of the broad-host-range lactococcal plasmid pWVO1", PLASMID, NEW YORK,NY, US, vol. 26, no. 1, 1 July 1991 (1991-07-01), pages 55-66, XP024799763, ISSN: 0147-619X, DOI: 10.1016/0147-619X(91)90036-V [retrieved on 1991-07-01]
- BRUNSCHWIG E ET AL: "A two-component T7 system for the overexpression of genes in Pseudomonas aeruginosa", GENE, ELSEVIER, AMSTERDAM, NL, vol. 111, no. 1, 1 February 1992 (1992-02-01), pages 35-41, XP023542544, ISSN: 0378-1119, DOI: 10.1016/0378-1119(92)90600-T [retrieved on 1992-02-01]
- J Bacteriol ET AL: "Inducible cell lysis system for the study of natural transformation and environmental fate of DNA released by cell death.", , 1 January 1994 (1994-01-01), page 7352, XP055097413, Retrieved from the Internet: URL:http://jb.asm.org/content/176/23/7352. full.pdf [retrieved on 2014-01-20]
- TRINE AAKVIK ET AL: "A plasmid RK2-based broad-host-range cloning vector useful for transfer of metagenomic libraries to a variety of bacterial species", FEMS MICROBIOLOGY LETTERS, vol. 296, no. 2, 1 July 2009 (2009-07-01), pages 149-158, XP055097499, ISSN: 0378-1097, DOI: 10.1111/j.1574-6968.2009.01639.x
- SEEGERS J F ET AL: "Structural and functional analysis of the single-strand origin of replication from the lactococcal plasmid pWV01.", MOLECULAR & GENERAL GENETICS : MGG 1 NOV 1995, vol. 249, no. 1, 1 November 1995 (1995-11-01), pages 43-50, XP001526529, ISSN: 0026-8925

## Description

### Background of the invention

The invention relates to a synthetic nucleic acid molecule for expressing at least one nucleotide sequence of interest in at least one prokaryotic host cell, comprising at least one promoter sequence and at least one cloning site for inserting the nucleotide sequence of interest, wherein the cloning site is located downstream of the promoter sequence, at least one replication module comprising at least one replication cassette for promoting replication of the nucleic acid molecule, at least one expression module for promoting expression of the nucleotide sequence of interest in the host cell, and at least one resistance module for providing the host cell with antibiotic resistance, wherein each module is flanked at either end by at least one unique restriction site.

### Prior art

The heterologous expression of genes in prokaryotes is challenging, especially if the genes originate from a distant host or if the source is uncertain, such as a metagenomic expression library. Many vectors have been developed based on broad host range origins of replication, but these all focus on either Gram(+) or Gram(-) prokaryotes.

*Escherichia coli* is the working horse in biotechnology for decades. But with the shifting focus in biotechnology to functional metagenomics, the expression of environmental DNA (eDNA) in *E*. *coli* becomes a bottleneck. One may be able to optimize DNA sequences for the needs of *E. coli* and to generate novel *E. coli* strains, but this does not apply for the establishment of expression libraries with unknown DNA sequences, such as eDNA. That prevents us from harnessing the enormous biotechnological potential of genomes from uncultured microorganisms. However some approaches have already been followed to circumvent *E. coli* as an expression host, such as the establishment of a metagenomic expression library in *Cupriavidus metallidurans*, *Streptomyces* spp., and *Pseudomonas fluorescens.* Although some of these approaches are based on broad-host range expression vectors, the number of hosts is very limited due to the focus on either Gram(+) or Gram(-) organisms.

Today's broad host vectors are mainly based on IncaP, RK2 or rolling circle replicating (RCR) plasmids like pCI411. A very frequently used RCR-plasmid is pGK12 from *Kluyveromyces lactis* CBS 2359, which can replicate in *E*. *coli* and mainly in Gram(+) organisms like *Bacillus subtilis*, *Borrelia burgdorferi* and *Lactococcus lactis.* Although its RCR origin is used in over 20 shuttle vectors, it has numerous disadvantages like its big size and instabilities in any host. Due to this poor performance it was never widely adopted and alternatives are of great interest.

Belev et al. [28] provided an expression vector for optimization of transcription and translation in *E. coli,* which allows the exchange of its major components using unique restriction sites. The vector contains a repressor gene/promotor module, fd terminator sequences, a f1 origin of replication, and an antibiotic resistance module.

WO 2007/038276 A2 discloses a group of modular cloning vectors for the synthesis of a transgene DNA construct for the purpose of gene expression or analysis of gene expression. A primary vector contains at least one multiple cloning site and multiple sets of rare restriction and/or unique homing endonuclease sites arranged in a linear pattern. This arrangement defines a modular architecture that allows the user to place inserts into a transgene construct without disturbing the integrity of DNA elements already incorporated into the primary vector in previous cloning steps.

US 2007/0059768 A1 discloses stable vectors for genomic library construction useful in Gram negative species, which contain the pBBR1 replicon that is capable of stable replication in a broad range of Gram negative species. The plasmid vectors contain bidirectional, rho-independent transcriptional terminators flanking the multiple cloning site. Each vector may vary in its selection marker region, mobilization function, and promoter used to express insert sequences.

Prior et al. [29] have provided a modular set of broad-host-range expression vectors for protein expression in Gram negative bacteria. These vectors use the broad-host-range pBBR1 replicon as well as the arabinose-inducible PBAD promoter and are available with six different antibiotic resistance cassettes.

Pérez-Arellano et al. [30] constructed shuttle cloning vectors that carry replication origins which are functional in *Escherichia coli* (p15A, pWV01, ColE1), *Lactococcus lactis*, lactobacilli, and *Bacillus subtilis* (pAMbeta1, pWV01) and thus can be used in a broad host range. These plasmids contain the lacZ-T1T2 cassette from pJDC9, which allows the X-gal selection and cloning of DNA fragments that could cause plasmid instability in *E. coli.* Furthermore, the antibiotic resistance markers (beta-lactamase, chloramphenicol acetyl transferase, and erythromycin transacetylase) and the theta and rolling circle replicating origins have been combined to obtain this set of compatible plasmids (plA family) that can be co-transformed, both in lactic acid bacteria and in *E. coli.*

Leenhouts et al. [31] determined the nucleotide sequence of the *Lactococcus lactis* broad-host-range plasmid pWVO1, replicating in both gram-positive and gram-negative bacteria. Thes analysis revealed four open reading frames (ORFs). ORF A appeared to encode a trans-acting 26.8-kDa protein (RepA), necessary for replication. The ORF C product was assumed to play a regulatory role in replication. Both RepA and the ORF C product showed substantial sequence similarity with the Rep proteins of the streptococcal plasmid pLS1. In addition, the plus origin of replication was identified on the basis of strong similarity with the plus origin of pLS1.

It is an objective of the present invention to provide a broad-host range expression vector for establishing expression libraries with unknown DNA sequences, such as environmental DNA.

### Summary of the invention

The objective is met by a synthetic nucleic acid molecule, wherein a replication cassette for Gram-negative organisms comprising a pBBR1 origin of replication and a replication cassette for Gram-positive organisms comprising a pWV01 origin of replication are provided. The nucleic acid molecule according to the invention represents a completely synthetic expression vector based on different origins of replication so as to allow for using various Gram(+) and Gram(-) hosts at the same time for expression. This also makes it easy to generate environmental DNA (eDNA) expression libraries for the functional screening in diverse hosts without focusing on either Gram(+) or Gram(-) organisms. Thus, this new tool may allow us to find novel biocatalysts, which were not functional in the limited number of vector compatible hosts so far. Moreover, the modular design of the nucleic acid molecule according to the invention allows for constructing diverse expression vectors which are each optimized for their intended use. To this end, each module of the nucleic acid molecule is flanked at either end by at least one unique restriction site so that each module may be replaced by another module having a different function and/or effect. This measure makes it easy to combine different modules such that the resulting expression vector is perfectly adapted to its intended application.

In an embodiment of the present invention the replication cassette for Gram-negative organisms can, for example, comprise the nucleotide sequence according to SEQ ID NO: 1.

In an embodiment of the present invention the replication cassette for Gram-positive organisms can comprise a modified pWV01 origin of replication comprising the nucleotide sequence according to SEQ ID NO: 2. This sequence represents the modified pWV01 which is optimized for use in the nucleic acid molecule according to the invention.

The pWV01 origin of replication of *Lactococcus lactis* subsp. *cremoris* Wg2 is much smaller than the RCR origin of pGK12 and seems to have a higher performance. The pBBR1 origin of replication of *Bordetella bronchiseptica* S87 is widely used and compatible with IncP, IncQ and IncW group plasmids as well as with ColE1 and p15A containing plasmids. While the pBBR1 mode of replication is unclear, pWV01 replicates via the rolling circle mechanism. However, it is surprising that these origins are compatible and can be placed on the same vector without any interference. It is therefore an advantageous aspect of the invention that pBBR1 and the optimized pWV01 may be combined on the same completely synthesized vector.

In an embodiment of the present invention the unique restriction site can, for example, be selected from the group consisting of BgIII and NotI. However, it is also possible to use other unique restriction sites as long as the modular character of the nucleic acid molecule according to the invention is maintained. The nucleic acid molecule according to the present invention can, for example, further comprise at least one transcription termination sequence located downstream of the cloning site, for example, a transcription termination sequence selected from the group consisting of SEQ ID NO: 3 (new_Ter), SEQ ID NO: 4 (T7_Ter), SEQ ID NO: 5 (trpA_Ter), and SEQ ID NO: 6 (t500_Ter).

While an initially developed vector was able to replicate solely in *Escherichia coli* Stbl2, a specialized strain used to house unstable inserts containing repetitive sequences, deletion of some of its terminator sequences and having the common *E. coli* strain DH5α select which of the remaining terminators were compatible with stable replication and thus the maintenance of a full-length vector, resulted in the expression vector according to the invention. Surprisingly, this strain not only preferred a specific set of terminators (SEQ ID NO: 4, SEQ ID NO: 5, and SEQ ID NO: 6) but also generated a completely novel one (SEQ ID NO: 3).

In an embodiment of the present invention the expression module can, for example, comprise a promoter sequence. For example, the promoter sequence can comprise a P*tac* promoter sequence (SEQ ID NO: 7).

In an embodiment of the present invention the expression module can, for example, comprise at least one regulatory sequence, for example, a *lac*I cassette and a *lac* operator sequence. In an embodiment of the present invention, the expression module can, for example, comprise the cloning site, for example, a multiple cloning site.

In an embodiment of the present invention the resistance module can, for example, comprise a chloramphenicol acetyl transferase (CAT) resistance cassette.

For example, the synthetic nucleic acid molecule according to the invention can, comprise at least one nucleotide sequence selected from the group consisting of:
a) a nucleotide sequence which comprises the nucleotide sequences of SEQ ID NO: 1, SEQ ID NO: 2, SEQ ID NO: 3, SEQ ID NO: 4, SEQ ID NO: 5, SEQ ID NO: 6, and SEQ ID NO: 7;
b) a nucleotide sequence according to SEQ ID NO: 8;
c) a nucleotide sequence, the complementary strand of which hybridizes with the nucleotide sequences of a) or b) under stringent conditions;
d) a nucleotide sequence which has at least 90% or 95%, identity with the nucleotide sequence of a), b) or c); and
e) a nucleotide sequence which corresponds to the complementary strand of the nucleotide sequence of a) to d).

The invention further concerns a prokaryotic cell including the nucleic acid molecule according to the invention as described above.

The invention also relates to a cell culture comprising at least one cell according to the invention.

A further aspect of the invention is the use of the nucleic acid molecule according to the invention as described above for heterologous expression of metagenomic expression libraries in at least one prokaryotic host cell, for example, for functional screening of environmental expression libraries. The nucleic acid molecule according to the invention can, for example, also be used for generating cDNA expression libraries of eukaryotic genes or introducing recombination cassettes for promoting specific knockouts or accelerating chromosomal localization.

Another aspect of the invention relates to a method for expressing at least one nucleotide sequence of interest in at least one prokaryotic host cell, said method comprising the following steps:
- inserting the nucleotide sequence of interest into a cloning site of the nucleic acid molecule according to the invention as described above;
- introducing the nucleic acid molecule into a host cell;
- cultivating the host cell under conditions that allow expression of the nucleotide sequence of interest, wherein the nucleotide sequence of interest is part of a metagenomic library.

According to the invention environmental DNA (eDNA) expression libraries can be easily generated and used for the functional screening in diverse hosts without focusing on either Gram(+) or Gram(-) organisms.

The term "heterologous expression" as used herein refers to a process wherein a gene or gene fragment is expressed in a host organism which does not naturally have this gene or gene fragment.

The term "metagenomic library" as used herein refers to a pool of genetic material recovered directly from environmental material comprising largely unbiased samples of all genes from all the members of the material. "Environmental material" may include but is not limited to environmental DNA (eDNA).

The term "nucleic acid" as used herein refers to a polymeric molecule comprising a consecutive sequence of nucleotide monomers ("nucleotides"). A nucleic acid molecule according to the invention may include but is not limited to deoxyribonucleic acid (DNA), ribonucleic acid (RNA), and nucleic acid analogs such as peptide nucleic acids (PNA).

The term "replication module" as used herein refers to a consecutive sequence of nucleotides comprising at least one genetic element that is necessary to propagate a nucleic acid molecule comprising said consecutive sequence of nucleotides by producing at least one identical copy of said nucleic acid molecule in a living cell. Herein, the genetic element may include but is not limited to an "origin of replication" which is a particular sequence that is specifically recognized and bound by a protein complex in order to initiate the replication process.

The term "expression module" as used herein refers to a consecutive sequence of nucleotides comprising at least one genetic element that is suitable for performing a process by which information from a gene is used for the synthesis of a functional gene product in a living cell. Herein, the genetic element may include but is not limited to promoter and regulatory sequences.

The term "resistance module" as used herein refers to a consecutive sequence of nucleotides comprising at least one genetic element that is suitable for providing a living cell with resistance against a specific antibiotic.

The term "restriction site" as used herein refers to a consecutive sequence of nucleotides which is specifically recognized by a specific restriction enzyme that is able to cut a nucleic acid sequence between two nucleotides within said restriction site, or somewhere nearby.

The invention is further exemplarily described in detail with reference to the figures.

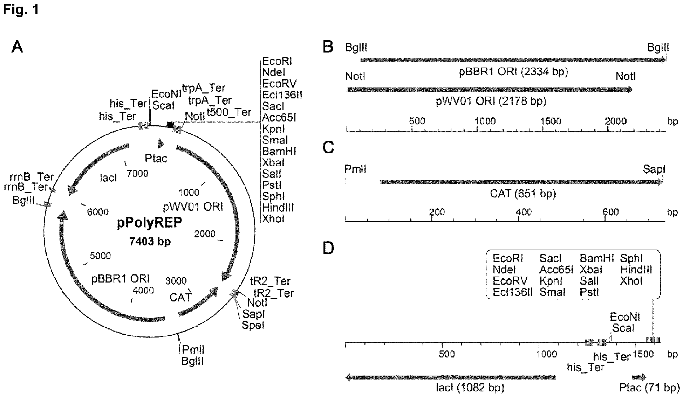

**Figure 1****: Generation of pPolyREP.** This figure shows the structure of an initially constructed vector called pPolyREP comprising four major modules: the replication modules pBBR1 (SEQ ID NO: 1) and pWV01 (B); the resistance module containing a chloramphenicol acetyltransferase cassette (CAT) (SEQ ID NO: 9) (C); and the expression module containing a *lac*I cassette (SEQ ID NO: 10), a P*tac* (SEQ ID NO: 7) promoter, a *lac* operator (SEQ ID NO: 11) and a multiple cloning site (D). This initially produced vector already has the modular character according to the invention, i.e. each module is flanked by unique restriction sites (B, C, D) and can be removed or replaced easily.

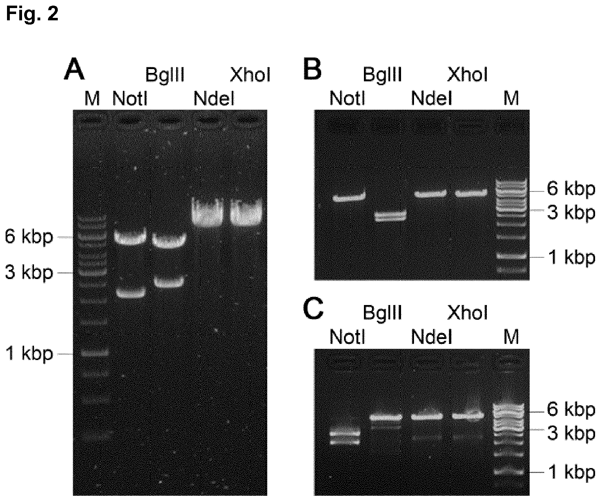

**Figure 2****: Analysis of pPolyREP (A) and the derivatives pPR_pBBR1 (B) and pPR_pWV01 (C).** This figure shows a gel electrophoretic analysis of the initially constructed vector pPolyREP. As becomes apparent, each origin of replication can be removed easily using either Notl, which removes pWV01 and generates pPR_pBBR1, or BgIII, which removes pBBR1 and generates pPR_pWV01 (See also Figure 1). While each origin can be removed from the full-length vector pPolyREP (Notl, 4901 + 1519 bp; BgIII, 3978 + 2442 bp, A), only the remaining origins in the derived vectors can be removed (BgIII, 2774 + 2442 bp, B; Notl, 2774 + 2187 bp, C). Isolation of the derivatives from *E*. *coli* Stbl2 clearly demonstrates the functionality of both individual origins. The Ndel and Xhol sites are part of the multiple cloning site and were used to linearize the vectors (7403 bp).

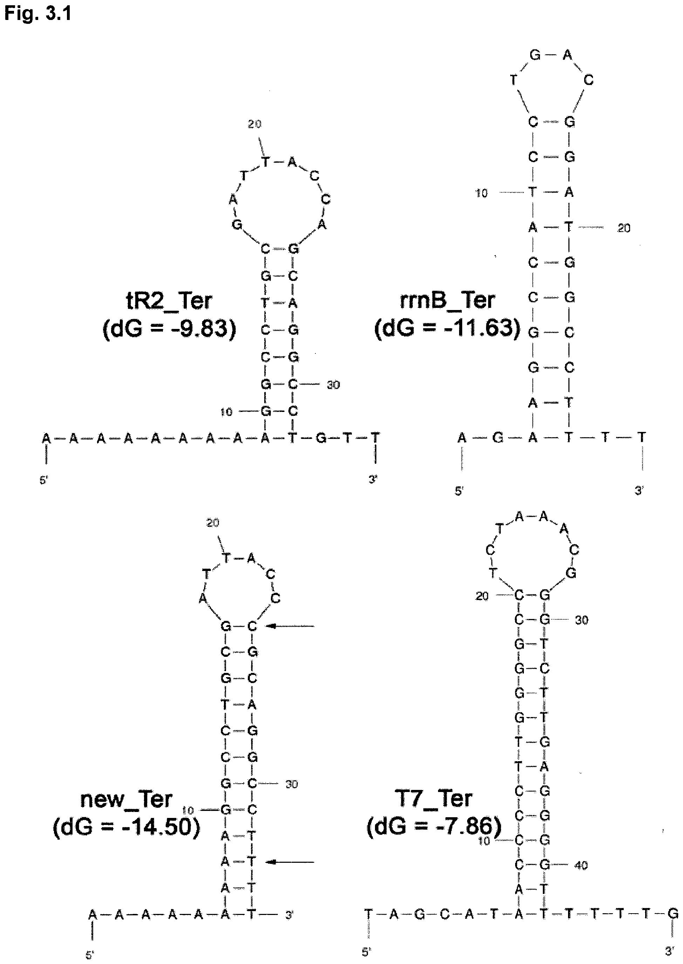

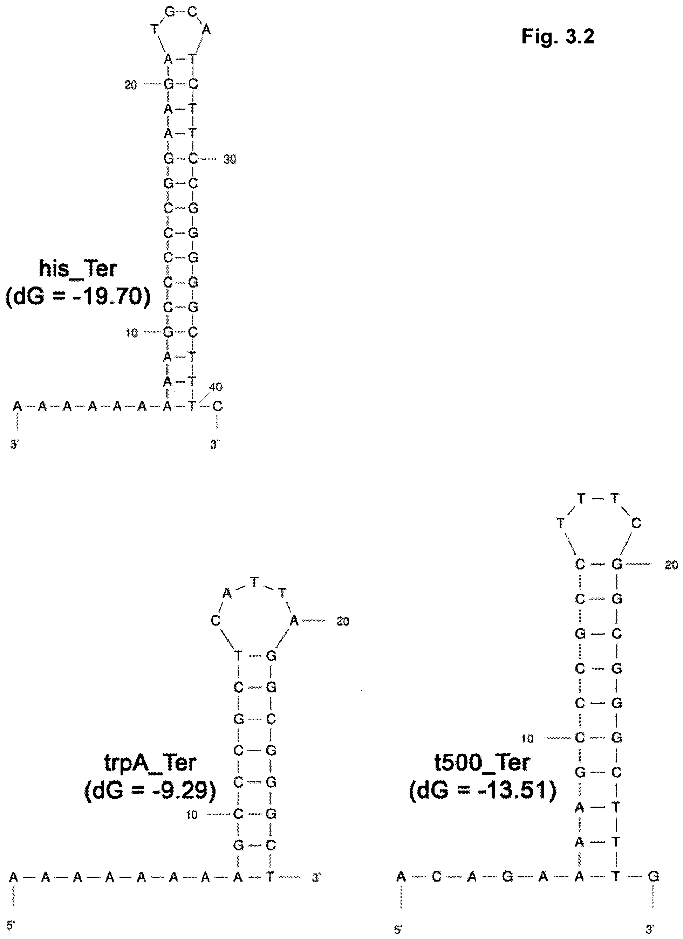

**Figure 3****: DNA sequences and calculated secondary structures of transcription terminators used.** This figure shows the structure of the transcription terminators according to the invention. The upper lane represents the transcription terminators present in pPolyREP, which were replaced, modified or deleted in one embodiment of a vector according to the invention (pPolyREPII). The lower lane represents the set of terminators, which were used in the final vector pPolyREPII. The difference in the calculated Gibbs free energy between the original tR2 terminator and the newly-generated terminator (new_Ter; SEQ ID NO: 3) is almost half that of tR2. This reflects the presence of two point mutations, marked with arrows, which reduce the size of the terminator loop but produce a longer stem and thus make it stronger than the original. The structure and free Gibbs energy were calculated with the mfold program.

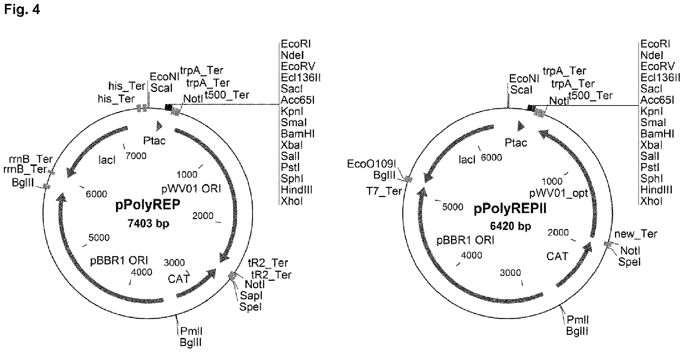

**Figure 4****: Modification of pPolyREP to generate an improved vector according to the invention pPolyREPII**. This figure shows the structures of the initially constructed vector pPolyREP and an improved vector according to the invention, pPolyREPII (SEQ ID NO: 8). To generate an optimized shuttle vector that can replicate in common *E. coli* strains such as DH5α, we removed the dual histidine terminators (his_Ter) and replaced the dual rrnB terminators (rrnB_Ter) with a T7 terminator (T7_Ter; SEQ ID NO: 4). Apparently, *E. coli* DH5α still experienced difficulties with the dual tR2 terminators (tR2_Ter) as the vector recovered from the transformed strains contained only a single copy of tR2_Ter, which was even modified to form a new terminator (new_Ter; SEQ ID NO: 3, Fig.3). These optimizations made it possible to transform *E. coli* DH5α with the new vector, which was maintained in this host in its original form. Later, we also optimized the origin pWV01 by truncation and reversing its orientation (Modified pWV01; SEQ ID NO: 2). These optimizations led to the final version of an exemplary vector according to the invention, pPolyREPII.

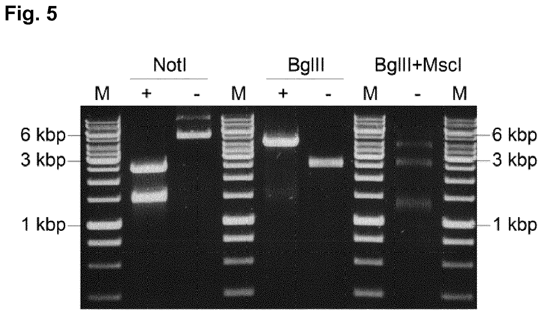

**Figure 5****: Analysis of the derivatives of pPolyREPII, pPolyREPII(+) and pPolyREPII(-) (A); and the structure of the original and new transcription terminators (B).** This figure shows a gel electrophoretic analysis of derivatives of pPolyREPII, wherein pPolyREPII(+) and pPolyREPII(-) are derivatives of pPolyREPII containing only the pWV01 or pBBR1 origins, respectively. While pWV01 can be removed from pPolyREPII(+) with NotI (2459 + 1519 bp), this vector is linearized by BgIII (3978 bp) due to the missing pBBR1 origin. Similarly, whereas pBBR1 can be removed from pPolyREPII(-) with BgIII (2459 + 2442 bp), this vector is linearized by Notl (4901 bp) due to the missing pWV01 origin. The digestion of pPolyREPII(-) with BgIII generates two fragments almost equal in size. Therefore we also digested pPolyREPII(-) with McsI in addition to BgIII, which cuts pBBR1 into almost equal-sized fragments (1267 + 1175 bp) and leaves the second fragment of the BgIII digest untouched (2459 bp).

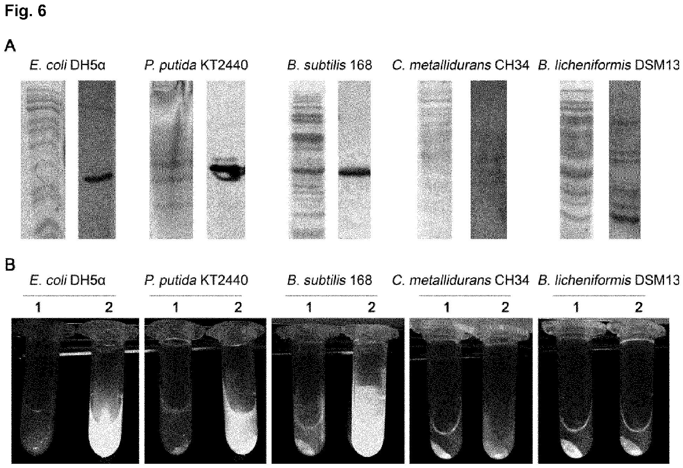

**Figure 6****: Expression studies with pPolyREPII and pPRII::GFP-His₆**. This figure shows a SDS-PAGE (left) and corresponding western blot with chemiluminescence detection using Anti-Penta-His IgG1 HRP conjugate (Qiagen, Germany) (right) of the crude extracts of each host transformed with pPRII:GFP-His₆ (A). The band detected in the crude extracts of *E. coli* DH5α, *P. putida* KT2440 and *B. subtilis* 168 corresponds to the molecular mass of GFP-His₆ (∼29.6 kDa). Each host was transformed with the empty vector pPolyREPII (1) and pPRII:GFP-His₆ (2) and the synthesis of GFP-His₆ was observed on a transilluminator (Blue LED Illuminator, excitation wavelength of 470 nm; NIPPON Genetics EUROPE GmbH, Germany) (B).

### Examples

### Chemicals

Restriction enzymes, the Rapid DNA ligation kit, and Phusion DNA polymerase were purchased from Fermentas (Thermo Fisher Scientific, St. Leon-Rot, Germany). Gibson Assembly™ Master Mix was purchased from New England Biolabs (Ipswitch, USA).

### Molecular genetics

The initial shuttle vector pPolyREP was completely synthesized by Geneart® (Thermo Fisher Scientific, St. Leon-Rot, Germany). The first modification was the removal of the tandem histidin terminator sequences (his-Ter) by a PCR with 5'-phosphorylated primers (pPR-HisT_1 & 2), followed by religation and transformation of *E*. *coli* DH5α. To rebuild the full-length vector starting from the isolated, truncated one, we conducted a Gibson Assembly [1] according to the manufacturer's manual with three amplificates. The first fragment resembled the original vector backbone with the newly generated terminator (new_Ter (SEQ ID NO: 3); primer pair GA_pPR_1 & 2), while the other two fragments resembled the missing *lac*I gene (primer pair GA_pPR_3 & 4) and the missing pBBR1 origin of replication (primer pair GA_pPR_5 & 6), respectively. By the last two PCRs we also replaced the tandem rrnB terminator sequence (rrnR_ter) with a T7 terminator sequence (T7_ter; SEQ ID NO: 4) in the overlapping region of the fragments. And finally we modified the pWV01 origin of replication according to Bryksin & Matsumura (2010) by an amplification with the primer pairs pWV01_opt_1 & 2 and insertion in the shuttle vector via Notl, eventually generating pPolyREPII. For expression trials, the *gfp* gene was amplified from pP_{T7}-GFP (MoBiTec GmbH, Göttingen, Germany) with the primer pair GFP_for (5'-phosphorylated) and GFP_His6_rev and ligated to pPolyREPII via EcoRV and Xbal. This cloned gene encodes GFP-His₆. All primer sequences mentioned here can be found in table 1. The correct sequences of all isolated vectors and inserts were confirmed by sequencing at MWG-Biotech AG (Ebersberg, Germany).

### Bacterial strains, transformation and culture conditions

*Escherichia coli* Stbl2 [2] was purchased from Life Technologies Corporation (Thermo Fisher Scientific, St. Leon-Rot, Germany) and used to harbor pPolyREP and derivatives. *E. coli* DH5α [3] was used as a host for pPolyREPII and derivatives. *Pseudomonas putida* KT2440 [4] and *Bacillus subtilis* 168 [5,6] were kindly provided by Prof. Dr. Susanne Fetzner (University of Münster, Germany). *B. licheniformis* DSM13 [7] and *Cupriavidus metallidurans* CH34 [8,9] were purchased from DSMZ (German Collection of Microorganisms and Cell Cultures, Braunschweig, Germany). The generation of competent cells, their transformation and selection with different concentrations of chloramphenicol is summarized in table 2. All strains harboring pPolyREP and pPolyREPII including their derivatives were grown in LB at 30°C with the corresponding concentration of chloramphenicol. Autoinduction solutions M (50× stock: 1.25 M Na₂HPO₄, 1.25 M KH₂PO₄, 2.5 M NH₄Cl, 0.25 M Na₂SO₄) and 5052 (50× stock: 25% (v/v) glycerol, 2.5% (w/v) D-glucose, 10% (w/v) α-lactose monohydrate) [10] were added to induce the cells for expression of *gfp.* Four hours prior to cell harvest, the expression was additionally induced by the addition of 0.5 mM isopropyl β-D-1-thiogalactopyranoside (IPTG).

### Protein analysis

Denaturing sodium dodecyl sulfate-polyacrylamide gel electrophoresis (SDS-PAGE) was performed as described by Laemmli [11], using an overall acrylamide concentration of 12% and a cross-linker concentration of 2.6% in the separating gels. Polyacrylamide gels were stained with Coomassie blue R-250 (0.1 % (w/w) Coomassie blue R-250, 50% (w/w) trichloroacetic acid in H₂O) and destained in an aqueous solution of 30% (v/v) methanol and 10% (v/v) acetic acid. Transfer of proteins [12] from gels to nitrocellulose membranes (GE Healthcare Europe GmbH, Freiburg, Germany) was performed according to the protocol of QIAGEN (QIA*express*). Immunodetection of His₆-tagged proteins on blots was performed using Anti-Penta-His IgG1 HRP conjugate (Qiagen, Germany) and detection by chemiluminescence. GFP-His₆ synthesis in the crude extracts was visualized with a transilluminator (Blue LED Illuminator, excitation wavelength of 470 nm; NIPPON Genetics EUROPE GmbH, Germany).

### Generation of the initial shuttle vector pPolyREP

The shuttle vector was initially divided into functional subunits to facilitate the generation of defined modules that could be easily exchanged or deleted (Fig.1). The first module was a replication module (Fig.1 B) based on the pBBR1 cassette, which is derived from *Bordetella bronchiseptica* S87. This origin of replication is known to replicate in a broad range of Gram(-) organisms [13,14] and is used in diverse vector systems. The second replication module was pWV01, which is derived from a cryptic plasmid found in *Lactococcus lactis* subsp. *cremoris* Wg2. Although this origin also replicates in *E*. *coli,* it predominantly replicates in Gram(+) organisms [31].

The third module was a chloramphenicol acetyl transferase (CAT) resistance cassette (Fig.1 C), comprising a common constitutive promoter followed by the CAT gene; this cassette can be found in many prokaryotes and thus is known to be functional in many different hosts [17,18].

The fourth module was the expression module, consisting of a *lac*I cassette and an improved hybrid of the common *lac* UV5 promoter (P*lac*) and *trp* promoter (P*trp*) known as P*tac* [19] (SEQ ID NO: 7). This was followed by a *lac* operator and a multiple cloning site. The P*tac* promoter is approximately seven times stronger than the common P*lac* promoter, but is still compatible with a broad range of Gram(+) and Gram(-) hosts.

The introduction of transcription terminators can promote vector stability in different hosts [15,20]. Therefore, we introduced a series of different transcription terminators (for an overview see: [21]) downstream of each gene and the multiple cloning site. The complete synthesis and assembly of the full-length shuttle vector was carried out by GeneArt® (Thermo Fisher Scientific, St. Leon-Rot, Germany).

### Evaluation of pPolyREP

The initial shuttle vector pPolyREP was propagated solely by the specialized E. *coli* strain Stbl2, which unlike the common strain DH5α can maintain DNA sequences containing unstable repeats [2]. A *gfp* gene with a downstream His₆ tag sequence was cloned in this vector, generating pPR::GFP-His₆. *E. coli* Stbl2, *B. subtilis* 168, *B. licheniformis* DSM13, *C. metallidurans* CH34 and *P. putida* KT2440 were each transformed separately with pPolyREP and pPR::GFP-His₆.

Although we were able to select resistant transformants for all strains, GFP-His₆ was only synthesized in *E. coli* Stbl2 and *P. putida* KT2440 (data not shown). This suggested that the other strains may have experienced problems similar to those reported in *E. coli* DH5α, reflecting the presence of multiple terminator sequences. It was also unclear whether pWV01 was functional, because only Gram(-) organisms were able to synthesize GFP-His₆.

We therefore exploited the modular design of the shuttle vector and removed either of the two origins of replication by cutting the vector with BgIII and NotI, generating pPR_pBBR1 and pPR_pWV01, respectively (Fig.2). We transformed *E. coli* Stbl2 with both of these vectors, and plasmid DNA isolated from the transformants revealed the vectors were recovered unchanged, with single origins as anticipated (Fig.2B&C).

### Optimization of pPolyREP to pPolyREPII

Our initial hypothesis explaining the failure of the shuttle vector to replicate in common *E. coli* strains was the large number and tandem organization of the terminator sequences. Analysis of the terminator regions in pPolyREP using the mfold program [22] showed that the tandem histidine terminators (his_Ter) and the tandem tryptophan terminators (trpA_Ter; SEQ ID NO: 5) were the strongest terminator regions, based on their calculated high Gibbs free energy values [23].

We therefore used PCR to delete the corresponding regions from pPolyREP and introduced the derived vectors into *E. coli* DH5α, which was unable to maintain the original vector. We recovered transformants containing the vector derivative with the histidine terminator sequences deleted but the tryptophan terminators remaining intact, indicating that the host experienced difficulty with the tandem histidine terminators. However the sequence of the isolated vector revealed additional truncations in the region between *lac*I and pBBR1, suggesting that the tandem rrnB terminators were also problematic. Interestingly, the tandem tR2 terminators (tR2_Ter) were also modified by the partial deletion of one copy and the introduction of two point mutations. This generated a completely new terminator (new_Ter; SEQ ID NO: 3) as confirmed with the mfold program (Fig.3). To complement the missing parts, we carried out a Gibson assembly with three parts, directly replacing the dual rrnB terminator with a T7 terminator (T7_Ter; SEQ ID NO: 4). The resulting vector was maintained in *E. coli* DH5α at full length. To prevent problems potentially caused by an unstable pWV01 origin, we also truncated that sequence according to Bryksin and Matsumura (2010) [16] and inserted pWV01_opt (Modified pWV01 ori; SEQ ID NO: 2) into the NotI site in the opposite orientation, yielding the final shuttle vector, pPolyREPII (Fig.4).

### Evaluation of pPolyREPII

The full-length vector pPolyREPII was digested with BgIII or NotI to generate the derivatives pPolyREP(+) and pPolyREP(-), which carry only the pWV01 or pBBR1 origins, respectively. Digesting these derivatives with BgIII or Notl or BgIII and MscI produced fragments of the anticipated sizes (Fig.5). We also sequenced pPolyREPII and both derivatives to verify the expected sequences. The expression capabilities of pPolyREPII were evaluated by inserting a *gfp* gene with a downstream His₆ tag sequence, generating pPRII::GFP-His₆. *E. coli* Stbl2, *B. subtilis* 168, *B. licheniformis* DSM13, *C. metallidurans* CH34 and *P. putida* KT2440 were each transformed separately with pPolyREPII and pPRII::GFP-His₆. Following induction, crude extracts from each strain were analyzed by SDS-PAGE, western blot and GFP fluorescence (Fig.6). We observed strong GFP expression in *E. coli* DH5α, *Pseudomonas putida* KT2440, *Bacillus subtilis* 168, weak expression in *Cupriavidus metallidurans* CH34, and no expression in *Bacillus licheniformis* DSM13. The presence of the GFP-His₆ protein was confirmed by western blot.
Although we did not see an expression of *gfp* in *B. licheniformis* DSM13, it must not point to a failed transformation, especially because we were able to differentiate resistant transformats from those which were not resistant. However a problem may be based on the expression reporter, *gfp* itself, since it is the unmodified "wildtype" form, which is not optimized for expression in any of those hosts. This can be seen for the expression in *C. metallidurans* CH34, which is much weaker than that in the other hosts.

As a result, a very effective broad host range expression vector is provided, which can be equally well established in Gram(+) and Gram(-) hosts. This is possible by a rational design and using the recent advantages in synthetic biology. The novel nucleic acid molecule (expression vector) according to the invention can be used to establish eDNA expression libraries and to screen for desired activities in different Gram(+) as well as Gram(-) hosts at the same time.

**Table 1: Primer sequences used to generate pPolyREPII and derivatives.**

| **Primer** | **Sequence (5'→3')** |
|---|---|
| pPR-HisT_1 | PHO-CACCGTGCAGTCGATAAGC (SEQ ID NO: 12) |
| pPR-HisT_2 | PHO-GCTGTGGTATGGCCTGTG (SEQ ID NO: 13) |
| GA_pPR_1 | CACATTCACCACCCTGAATTGAC (SEQ ID NO: 14) |
| GA_pPR_2 | GATCTCACGTGGGATTGATTCTAATG (SEQ ID NO: 15) |
| GA_pPR_3 | |
| GA_pPR_4 | GTCAATTCAGGGTGGTGAATGTG (SEQ ID NO: 17) |
| GA_pPR_5 | CATTAGAATCAATCCCACGTGAGATC (SEQ ID NO: 18) |
| GA_pPR_6 | |
| pWV01_opt_1 | |
| pWV01_opt_2 | |
| GFP_for | PHO-ATGGTCCAAACTAGTTCGAAGATC (SEQ ID NO: 22) |
| GFP_His6_rev | |

**Table 2: Transformation and selection of each strain. After transformation each strain was regenerated with SOC medium (Life Technologies Corporation) for 2 h at 30°C before plating out on LB selection agar.**

| **Strain** | **Method of transformation** | **Reference for transformation** | **Chloramphenicol (µg/ml)** |
|---|---|---|---|
| *B. subtilis* 168 | Electroporation | [24] | 15 |
| *B. licheniformis* DSM13 | Electroporation | [24] | 15 |
| *C. metallidurans* CH34 | Electroporation | [25] | 150 |
| *E. coli* Stbl2 | Heat shock | [26] | 17 |
| *E. coli* DH5α | Heat shock | [26] | 17 |
| *P. putida* KT2440 | Electroporation | [27] | 250 |

### Literature

1. Gibson D, Young L, Chuang R, Venter J, Hutchison III C, et al. (2009) Enzymatic assembly of DNA molecules up to several hundred kilobases. Nature Methods 6: 343-345. Available: http://www.nature.com/nmeth/journal/vaop/ncurrent/full/nmeth.1318.html. Accessed 2 May 2013.
2. Trinh T, Jessee J, Bloom F, Hirsch V (1994) STBL2: an Escherichia coli strain for the stable propagation of retroviral clones and direct repeat sequences. Focus 16: 78-80.
3. Grant SG, Jessee J, Bloom FR, Hanahan D (1990) Differential plasmid rescue from transgenic mouse DNAs into Escherichia coli methylation-restriction mutants. Proceedings of the National Academy of Sciences of the United States of America 87: 4645-4649. Available: http://www.pubmedcentral.nih.gov/articlerender.fcgi?artid=54173&tool=p mcentrez&rendertype=abstract.
4. Nelson KE, Weinel C, Paulsen IT, Dodson RJ, Hilbert H, et al. (2002) Complete genome sequence and comparative analysis of the metabolically versatile Pseudomonas putida KT2440. Environmental microbiology 4: 799-808. Available: http://www.ncbi.nlm.nih.gov/pubmed/12534463.
5. Zeigler DR, Prágai Z, Rodriguez S, Chevreux B, Muffler A, et al. (2008) The origins of 168, W23, and other Bacillus subtilis legacy strains. Journal of bacteriology 190: 6983-6995. Available: http://www.pubmedcentral.nih.gov/articlerender.fcgi?artid=2580678&tool= pmcentrez&rendertype=abstract. Accessed 28 April 2013.
6. Barbe V, Cruveiller S, Kunst F, Lenoble P, Meurice G, et al. (2009) From a consortium sequence to a unified sequence: the Bacillus subtilis 168 reference genome a decade later. Microbiology (Reading, England) 155: 1758-1775. Available: http://www.pubmedcentral.nih.gov/articlerender.fcgi?artid=2885750&tool= pmcentrez&rendertype=abstract. Accessed 28 February 2013.
7. Veith B, Herzberg C, Steckel S, Feesche J, Maurer KH, et al. (2004) The complete genome sequence of Bacillus licheniformis DSM13, an organism with great industrial potential. Journal of molecular microbiology and biotechnology 7: 204-211. Available: http://www.ncbi.nlm.nih.gov/pubmed/15383718. Accessed 5 March 2013.
8. Vandamme P, Coenye T (2004) Taxonomy of the genus Cupriavidus: a tale of lost and found. International journal of systematic and evolutionary microbiology 54: 2285-2289. Available: http://www.ncbi.nlm.nih.gov/pubmed/15545472. Accessed 4 April 2013.
9. Vaneechoutte M (2004) Wautersia gen. nov., a novel genus accommodating the phylogenetic lineage including Ralstonia eutropha and related species, and proposal of Ralstonia [Pseudomonas] syzygii (Roberts et al. 1990) comb. nov. International Journal of Systematic and Evolutionary Microbiology 54: 317-327. Available: http://ijs.sgmjournals.org/cgi/doi/10.1099/ijs.0.02754-0. Accessed 1 March 2013.
10. Studier WF (2005) Protein Production by Auto-Induction in High-Density Shaking Cultures. Protein Expression and Purification 41: 207-234. Available: http://www.sciencemag.org/content/306/5703/1895.short. Accessed 2 May 2013.
11. Laemmli U (1970) Cleavage of structural proteins during the assembly of the head of bacteriophage T4. Nature 227: 680-685. Available: http://bioinfcpcri.org/misc/nature.pdf. Accessed 2 May 2013.
12. Towbin H, Staehelin T, Gordon J (1979) Electrophoretic transfer of proteins from polyacrylamide gels to nitrocellulose sheets: procedure and some applications. 1979. Proceedings of the National Academy of Sciences of the United States of America 76: 4350-4354. Available: http://www.ncbi.nlm.nih.gov/pubmed/1422008.
13. Prior JE, Lynch MD, Gill RT (2010) Broad-host-range vectors for protein expression across gram negative hosts. Biotechnology and bioengineering 106: 326-332. Available: http://www.ncbi.nlm.nih.gov/pubmed/20148414. Accessed 1 May 2013.
14. Lale R, Brautaset T, Valla S (2011) Broad-Host-Range Plasmid Vectors for Gene Expression in Bacteria. In: Williams JA, editor. Strain Engineering SE - 19. Humana Press, Vol. 765. pp. 327-343. Available: http://dx.doi.org/10.1007/978-1-61779-197-0_19.
15. Pérez-arellano I, Zúñiga M, Pérez-Martínez G (2001) Construction of compatible wide-host-range shuttle vectors for lactic acid bacteria and Escherichia coli. Plasmid 46: 106-116. Available: http://www.ncbi.nlm.nih.gov/pubmed/11591136. Accessed 26 April 2013.
16. Bryksin A V, Matsumura I (2010) Rational design of a plasmid origin that replicates efficiently in both gram-positive and gram-negative bacteria. PloS one 5: e13244. Available: http://www.pubmedcentral.nih.gov/articlerender.fcgi?artid=2951906&tool= pmcentrez&rendertype=abstract. Accessed 26 April 2013.
17. Schwarz S, Kehrenberg C, Doublet B, Cloeckaert A (2004) Molecular basis of bacterial resistance to chloramphenicol and florfenicol. FEMS microbiology reviews 28: 519-542. Available: http://www.ncbi.nlm.nih.gov/pubmed/15539072. Accessed 3 March 2013.
18. Murray IA, Shaw W V (1997) O-Acetyltransferases for Chloramphenicol and Other Natural Products. Antimicrobial Agents and Chemotherapy 41: 1-6.
19. Comstock J, de Boer HA, Comstock LJ, Vasser M (1983) The tac promoter: A functional hybrid derived from the trp and lac promoters. Biochemistry 80: 21-25. Available: http://www.pubmedcentral.nih.gov/articlerender.fcgi?artid=393301&tool=p mcentrez&rendertype=abstract.
20. Kieser T, Melton RE (1988) Plasmid pIJ699, a multi-copy positive-selection vector for Streptomyces. Gene 65: 83-91. Available: http://www.ncbi.nlm.nih.gov/pubmed/2840358.
21. Larson MH, Greenleaf WJ, Landick R, Block SM, Program B (2008) Applied force reveals mechanistic and energetic details of transcription termination. Cell 132: 971-982. Available: http://www.pubmedcentral.nih.gov/articlerender.fcgi?artid=2295211&tool= pmcentrez&rendertype=abstract. Accessed 11 March 2013.
22. Zuker M (2003) Mfold web server for nucleic acid folding and hybridization prediction. Nucleic Acids Research 31: 3406-3415. Available: http://nar.oxfordjournals.org/lookup/doi/10.1093/nar/gkg595. Accessed 28 February 2013.
23. SantaLucia J (1998) A unified view of polymer, dumbbell, and oligonucleotide DNA nearest-neighbor thermodynamics. Proceedings of the National Academy of Sciences of the United States of America 95: 1460-1465. Available: http://www.pubmedcentral.nih.gov/articlerender.fcgi?artid=19045&tool=p mcentrez&rendertype=abstract.
24. Xue G-P, Johnson JS, Dalrymple BP (1999) High osmolarity improves the electro-transformation efficiency of the gram-positive bacteria Bacillus subtilis and Bacillus licheniformis. Journal of Microbiological Methods 34: 183-191. Available: http://linkinghub.elsevier.com/retrieve/pii/S0167701298000876.
25. Taghavi S, van der Lelie D, Mergeay M (1994) Electroporation of Alcaligenes eutrophus with (Mega) Plasmids and Genomic DNA Fragments. Applied and Environmental Microbiology 60: 3585-3591.
26. Hanahan D (1983) Studies on transformation of Escherichia coli with plasmids. Journal of Molecular Biology 166: 557-580.
27. Iwasaki K, Uchiyama H, Yagi O, Kurabayashi T, Ishizuka K, et al. (1994) Transformation of Pseudomonas putida by Electroporation. Bioscience, Biotechnology, and Biochemistry 58: 851-854. Available: http://scholar.google.com/scholar?hl=en&btnG=Search&q=intitle:NII-Electronic+Library+Service#0.
28. Belev T N, Singh M, McCarthy J E G (1991) A fully modular vector system for the optimization of gene expression in Escherichia coli. Plasmid 26, 147-150.
29. Prior J E, Lynch M D, Gill R T (2010) Broad-host-range vectors for protein expression across Gram negative hosts. Biotechnology and Bioengineering, Vol. 106, No. 2, 326-332.
30. Pérez-Arellano I, Zúňiga M, Pérez-Martinez G (2001) Construction of compatible wide-host-range shuttle vectors for lactic acid bacteria and Escherichia coli. Plasmid 46, 106-116.
31. Leenhouts K J, Tolner B, Bron S, Kok J, Venema G, Seegers J F M L (1991) Nucleotide sequence and characterization of the broad-host-range lactococcal plasmid pWV01. Plasmid 26, 55-66.

## Claims

1. A synthetic nucleic acid molecule for expressing at least one nucleotide sequence of interest in at least one prokaryotic host cell, comprising at least one promoter sequence and at least one cloning site for inserting the nucleotide sequence of interest, wherein the cloning site is located downstream of the promoter sequence, at least one replication module comprising at least one replication cassette for promoting replication of the nucleic acid molecule, at least one expression module for promoting expression of the nucleotide sequence of interest in the host cell, and at least one resistance module for providing the host cell with antibiotic resistance, wherein each module is flanked at either end by at least one unique restriction site, **characterized in that** a replication cassette for Gram-negative organisms comprising a pBBR1 origin of replication and a replication cassette for Gram-positive organisms comprising a pWV01 origin of replication are provided.

2. The nucleic acid molecule according to claim 1, wherein the replication cassette for Gram-negative organisms comprises the nucleotide sequence according to SEQ ID NO: 1.

3. The nucleic acid molecule according to claim 1 or 2, wherein the replication cassette for Gram-positive organisms comprises a modified pWV01 origin of replication comprising the nucleotide sequence according to SEQ ID NO: 2.

4. The nucleic acid molecule according to any one of claims 1 to 3, wherein the unique restriction site is selected from the group consisting of BgIII and NotI.

5. The nucleic acid molecule according to any one of claims 1 to 4, further comprising at least one transcription termination sequence located downstream of the cloning site.

6. The nucleic acid molecule according to claim 5, wherein the transcription termination sequence is selected from the group consisting of SEQ ID NO: 3, SEQ ID NO: 4, SEQ ID NO: 5, and SEQ ID NO: 6.

7. The synthetic nucleic acid molecule according to any one of claims 1 to 6, comprising at least one nucleotide sequence selected from the group consisting of:
a) a nucleotide sequence which comprises the nucleotide sequences of SEQ ID NO: 1, SEQ ID NO: 2, SEQ ID NO: 3, SEQ ID NO: 4, SEQ ID NO: 5, SEQ ID NO: 6, and SEQ ID NO: 7;
b) a nucleotide sequence according to SEQ ID NO: 8;
c) a nucleotide sequence, the complementary strand of which hybridizes with the nucleotide sequences of a) or b) under stringent conditions;
d) a nucleotide sequence which has at least 90%, preferably 95%, identity with the nucleotide sequence of a), b) or c); and
e) a nucleotide sequence which corresponds to the complementary strand of the nucleotide sequence of a) to d).

8. A prokaryotic cell including the nucleic acid molecule according to any one of claims 1 to 7.

9. A cell culture comprising at least one cell according to claim 8.

10. Use of the nucleic acid molecule according to any one of claims 1 to 7 for heterologous expression of metagenomic expression libraries in at least one prokaryotic host cell, preferably for functional screening of environmental expression libraries.

11. Method for expressing at least one nucleotide sequence of interest in at least one prokaryotic host cell, **characterized by**
- inserting the nucleotide sequence of interest into a cloning site of the nucleic acid molecule according to any one of claims 1 to 7;
- introducing the nucleic acid molecule into a host cell;
- cultivating the host cell under conditions that allow expression of the nucleotide sequence of interest, wherein the nucleotide sequence of interest is part of a metagenomic library.

## Patentansprüche

1. Synthetisches Nukleinsäure-Molekül zur Expression mindestens einer Ziel-Nukleotid-Sequenz in mindestens einer prokaryontischen Wirtszelle, umfassend mindestens eine Promotor-Sequenz und mindestens eine Klonierungsstelle zum Einfügen der Ziel-Nukleotid-Sequenz, wobei die Klonierungsstelle stromabwärts von der Promotor-Sequenz angeordnet ist, mindestens ein Replikationsmodul, das mindestens eine Replikationskassette zum Voranbringen der Replikation des Nukleinsäure-Moleküls umfasst, mindestens ein Expressionsmodul zum Voranbringen der Expression der Ziel-Nukleotid-Sequenz in der Wirtszelle, und mindestens ein Resistenzmodul zum Versehen der Wirtszelle mit Antibiotikaresistenz, wobei jedes Modul an beiden Enden von mindestens einer einmaligen Restriktionsschnittstelle flankiert ist, **dadurch gekennzeichnet, dass** eine Replikationskassette für Gram-negative Organismen, die einen pBBR1-Replikationsursprung umfasst, und eine Replikationskassette für Gram-positive Organismen, die einen pWV01-Replikationsursprung umfasst, vorgesehen sind.

2. Nukleinsäure-Molekül nach Anspruch 1, wobei die Replikationskassette für Gram-negative Organismen die Nukleotid-Sequenz gemäß SEQ ID NO: 1 umfasst.

3. Nukleinsäure-Molekül nach Anspruch 1 oder 2, wobei die Replikationskassette für Gram-positive Organismen einen modifizierten pWV01-Replikationsursprung umfasst, der die Nukleotid-Sequenz gemäß SEQ ID NO: 2 umfasst.

4. Nukleinsäure-Molekül nach einem der Ansprüche 1 bis 3, wobei die einmalige Restriktionsschnittstelle aus der Gruppe ausgewählt ist, die aus BgIII und Notl besteht.

5. Nukleinsäure-Molekül nach einem der Ansprüche 1 bis 4, ferner umfassend mindestens eine Transkriptionsterminations-Sequenz, die stromabwärts von der Klonierungsstelle angeordnet ist.

6. Nukleinsäure-Molekül nach Anspruch 5, wobei die Transkriptionsterminations-Sequenz aus der Gruppe ausgewählt ist, die aus SEQ ID NO: 3, SEQ ID NO: 4, SEQ ID NO: 5 und SEQ ID NO: 6 besteht.

7. Synthetisches Nukleinsäure-Molekül nach einem der Ansprüche 1 bis 6, umfassend mindestens eine Nukleotid-Sequenz, die aus der Gruppe ausgewählt ist, bestehend aus:
a) einer Nukleotid-Sequenz, welche die Nucleotid-Sequenzen der SEQ ID NO: 1, SEQ ID NO: 2, SEQ ID NO: 3, SEQ ID NO: 4, SEQ ID NO: 5, SEQ ID NO: 6 und SEQ ID NO: 7 umfasst;
b) einer Nukleotid-Sequenz gemäß SEQ ID NO: 8;
c) einer Nukleotid-Sequenz, deren komplementärer Strang mit der Nukleotid-Sequenz gemäß a) oder b) unter stringenten Bedingungen hybridisiert;
d) einer Nukleotid-Sequenz, die mindestens 90%, vorzugsweise 95%, Identität mit der Nukleotid-Sequenz gemäß a), b) oder c) aufweist; und
e) einer Nukleotid-Sequenz, die dem komplementären Strang der Nukleotid-Sequenz gemäß a) bis d) entspricht.

8. Prokaryontische Zelle, die das Nukleinsäure-Molekül gemäß einem der Ansprüche 1 bis 7 beinhaltet.

9. Zellkultur, die mindestens eine Zelle gemäß Anspruch 8 umfasst.

10. Verwendung des Nukleinsäure-Moleküls nach einem der Ansprüche 1 bis 7 zur heterologen Expression von metagenomischen Expressionsbibliotheken in mindestens einer prokaryontischen Wirtszelle, vorzugsweise zum funktionellen Screening von Umwelt-Expressionsbibliotheken.

11. Verfahren zur Expression mindestens einer Ziel-Nukleotid-Sequenz in mindestens einer prokaryontischen Wirtszelle, **gekennzeichnet durch**
- Einfügen der Ziel-Nukleotid-Sequenz in eine Klonierungsstelle des Nukleinsäure-Moleküls nach einem der Ansprüche 1 bis 7;
- Einbringen des Nukleinsäure-Moleküls in eine Wirtszelle;
- Kultivieren der Wirtszelle unter Bedingungen, die eine Expression der Ziel-Nukleotid-Sequenz ermöglichen, wobei die Ziel-Nukleotid-Sequenz Teil einer metagenomischen Bibliothek ist.

## Revendications

1. Molécule d'acide nucléique synthétique destinée à exprimer au moins une séquence nucléotidique d'intérêt au sein d'au moins une cellule hôte procaryote, comprenant au moins une séquence promotrice et au moins un site de clonage pour insérer la séquence nucléotidique d'intérêt, où le site de clonage est situé en aval de la séquence promotrice, au moins un module de réplication comprenant au moins une cassette de réplication destinée à promouvoir la réplication de la molécule d'acide nucléique, au moins un module d'expression destiné à promouvoir l'expression de la séquence nucléotidique d'intérêt dans la cellule hôte, et au moins un module de résistance destiné à fournir à la cellule hôte une résistance antibiotique, où chaque module est entouré à l'une ou l'autre de ses extrémités d'au moins un site de restriction unique, **caractérisée en ce que** la cassette de réplication pour les organismes Gram-négatif comprenant une origine de réplication pBBR1 et une cassette de réplication pour les organismes Gram-positif comprenant une origine de réplication pWV01 sont fournies.

2. Molécule d'acide nucléique selon la revendication 1, où la cassette de réplication pour les organismes Gram-négatif comprend la séquence nucléotidique selon la SEQ ID NO:1.

3. Molécule d'acide nucléique selon les revendications 1 ou 2, où la cassette de réplication pour les organismes Gram-positif comprend une origine de réplication pWV01 modifiée comprenant la séquence nucléotidique selon la SEQ ID NO:2.

4. Molécule d'acide nucléique selon l'une quelconque des revendications 1 à 3, où le site de restriction unique est sélectionné dans le groupe comprenant les sites BgIII et Notl.

5. Molécule d'acide nucléique selon l'une quelconque des revendications 1 à 4, comprenant en outre une séquence de terminaison de la transcription située en aval du site de clonage.

6. Molécule d'acide nucléique selon la revendication 5, où la séquence de terminaison de la transcription est sélectionnée dans le groupe comprenant les SEQ ID NO:3, SEQ ID NO:4, SEQ ID NO:5 et SEQ ID NO:6.

7. Molécule d'acide nucléique selon l'une quelconque des revendications 1 à 6, comprenant au moins une séquence nucléotidique sélectionnée dans le groupe comprenant :
a) une séquence nucléotidique qui comprend les séquences nucléotidiques des SEQ ID NO:1, SEQ ID NO:2, SEQ ID NO:3, SEQ ID NO:4, SEQ ID NO:5, SEQ ID NO:6 et SEQ ID NO:7 ;
b) une séquence nucléotidique selon la SEQ ID NO:8 ;
c) une séquence nucléotidique dont le brin complémentaire s'hybride avec les séquences nucléotidiques de a) ou b) dans des conditions de stringence ;
d) une séquence nucléotidique possédant au moins 90 %, de préférence 95 %, d'identité commune avec la séquence nucléotidique de a), b) ou c) ; et
e) une séquence nucléotidique correspondant au brin complémentaire de la séquence nucléotidique de a) à d).

8. Cellule procaryote comprenant la molécule d'acide nucléique selon l'une quelconque des revendications 1 à 7.

9. Culture cellulaire comprenant au moins une cellule selon la revendication 8.

10. Utilisation de la molécule d'acide nucléique selon l'une quelconque des revendications 1 à 7 pour l'expression hétérologue de bibliothèques d'expression métagénomiques dans au moins une cellule hôte procaryote, de préférence pour un criblage fonctionnel des bibliothèques d'expression environnementales.

11. Procédé destiné à exprimer au moins une séquence nucléotidique d'intérêt dans au moins une cellule hôte procaryote, **caractérisé par** :
- l'insertion de la séquence nucléotidique d'intérêt dans un site de clonage de la molécule d'acide nucléique selon l'une quelconque des revendications 1 à 7 ;
- l'introduction de la molécule d'acide nucléique dans une cellule hôte ;
- la mise en culture de la cellule hôte dans des conditions permettant l'expression de la séquence nucléotidique d'intérêt, où la séquence nucléotidique d'intérêt fait partie d'une bibliothèque métagénomique.


### SEQUENCE LISTING

<110> Westfälische Wilhelms-Universität Münster
<120> Broad Host Range Expression Vector
<130> 13418 EP
<160> 23
<170> PatentIn version 3.5
<210> 1
   <211> 2334
   <212> DNA
   <213> Artificial Sequence
<220>
   <223> pBBR1 origin of replication
<400> 1
   tgcttgagac tggccgccac gttgcccatt ttcgccagct tcttgcatcg catgatcgcg      60
   tatgccgcca tgcctgcccc tcccttttgg tgtccaaccg gctcgacggg ggcagcgcaa     120
   ggcggtgcct ccggcgggcc actcaatgct tgagtatact cactagactt tgcttcgcaa     180
   agtcgtgacc gcctacggcg gctgcggcgc cctacgggct tgctctccgg gcttcgccct     240
   gcgcggtcgc tgcgctccct tgccagcccg tggatatgtg gacgatggcc gcgagcggcc     300
   accggctggc tcgcttcgct cggcccgtgg acaaccctgc tggacaagct gatggacagg     360
   ctgcgcctgc ccacgagctt gaccacaggg attgcccacc ggctacccag ccttcgacca     420
   catacccacc ggctccaact gcgcggcctg cggccttgcc ccatcaattt ttttaatttt     480
   ctctggggaa aagcctccgg cctgcggcct gcgcgcttcg cttgccggtt ggacaccaag     540
   tggaaggcgg gtcaaggctc gcgcagcgac cgcgcagcgg cttggccttg acgcgcctgg     600
   aacgacccaa gcctatgcga gtgggggcag tcgaaggcga agcccgcccg cctgcccccc     660
   gagcctcacg gcggcgagtg cgggggttcc aagggggcag cgccaccttg ggcaaggccg     720
   aaggccgcgc agtcgatcaa caagccccgg aggggccact ttttgccgga gggggagccg     780
   cgccgaaggc gtgggggaac ccgcaggggg tgcccttctt tgggcaccaa agaactagat     840
   atagggcgaa atgcgaaaga cttaaaaatc aacaacttaa aaaagggggg tacgcaacag     900
   ctcattgcgg caccccccgc aatagctcat tgcgtaggtt aaagaaaatc tgtaattgac     960
   tgccactttt acgcaacgca taattgttgt cgcgctgccg aaaagttgca gctgattgcg    1020
   catggtgccg caaccgtgcg gcaccctacc gcatggagat aagcatggcc acgcagtcca    1080
   gagaaatcgg cattcaagcc aagaacaagc ccggtcactg ggtgcaaacg gaacgcaaag    1140
   cgcatgaggc gtgggccggg cttattgcga ggaaacccac ggcggcaatg ctgctgcatc    1200
   acctcgtggc gcagatgggc caccagaacg ccgtggtggt cagccagaag acactttcca    1260
   agctcatcgg acgttctttg cggacggtcc aatacgcagt caaggacttg gtggccgagc 1320
   gctggatctc cgtcgtgaag ctcaacggcc ccggcaccgt gtcggcctac gtggtcaatg 1380
   accgcgtggc gtggggccag ccccgcgacc agttgcgcct gtcggtgttc agtgccgccg 1440
   tggtggttga tcacgacgac caggacgaat cgctgttggg gcatggcgac ctgcgccgca 1500
   tcccgaccct gtatccgggc gagcagcaac taccgaccgg ccccggcgag gagccgccca 1560
   gccagcccgg cattccgggc atggaaccag acctgccagc cttgaccgaa acggaggaat 1620
   gggaacggcg cgggcagcag cgcctgccga tgcccgatga gccgtgtttt ctggacgatg 1680
   gcgagccgtt ggagccgccg acacgggtca cgctgccgcg ccggtagcac ttgggttgcg 1740
   cagcaacccg taagtgcgct gttccagact atcggctgta gccgcctcgc cgccctatac 1800
   cttgtctgcc tccccgcgtt gcgtcgcggt gcatggagcc gggccacctc gacctgaatg 1860
   gaagccggcg gcacctcgct aacggattca ccgtttttat caggctctgg gaggcagaat 1920
   aaatgatcat atcgtcaatt attacctcca cggggagagc ctgagcaaac tggcctcagg 1980
   catttgagaa gcacacggtc acactgcttc cggtagtcaa taaaccggta aaccagcaat 2040
   agacataagc ggctatttaa cgaccctgcc ctgaaccgac gaccgggtcg aatttgcttt 2100
   cgaatttctg ccattcatcc gcttattatc acttattcag gcgtagcacc aggcgtttaa 2160
   gggcaccaat aactgcctta aaaaaattac gccccgccct gccactcatc gcagtcggcc 2220
   tattggttaa aaaatgagct gatttaacaa aaatttaacg cgaattttaa caaaatatta 2280
   acgcttacaa tttccattcg ccattcaggc tgcgcaactg ttgggaaggg cgat 2334
<210> 2
   <211> 1511
   <212> DNA
   <213> Artificial Sequence
<220>
   <223> Modified pWV01 origin of replication
<400> 2
   cgatttttta ttaaaacgtc tcaaaatcgt ttctgagacg ttttagcgtt tatttcgttt      60
   agttatcggc ataatcgtta aaacaggcgt tatcgtagcg taaaagccct tgagcgtagc     120
   gtggctttgc agcgaagatg ttgtctgtta gattatgaaa gccgatgact gaatgaaata     180
   ataagcgcag cgcccttcta tttcggttgg aggaggctca agggagtatg agggaatgaa     240
   attccctcat gggtttgatt ttaaaaattg cttgcatttt gccgagcggt agcgctggaa     300
   aatttttgaa aaaaatttgg aatttggaaa aaaatggggg gaaaggaagc gaattttgct     360
   tccgtactac gaccccccat taagtgccga gtgccaattt ttgtgccaaa aacgctctat     420
   cccaactggc tcaagggttt aaggggtttt tcaatcgcca acgaatcgcc aacgttttcg     480
   ccaacgtttt ttataaatct atatttaagt agctttattg ttgtttttat gattacaaag     540
   tgatacacta actttataaa attatttgat tggagttttt taaatggtga tttcagaatc     600
   gaaaaaaaga gttatgattt ctctgacaaa agagcaagat aaaaaattaa cagatatggc     660
   gaaacaaaaa ggtttttcaa aatctgcggt tgcggcgtta gctatagaag aatatgcaag     720
   aaaggaatca gaacaaaaaa aataagcgaa agctcgcgtt tttagaagga tacgagtttt     780
   cgctacttgt ttttgataag gtaattatat catggctatt aaaaatacta aagctagaaa     840
   ttttggattt ttattatatc ctgactcaat tcctaatgat tggaaagaaa aattagagag     900
   tttgggcgta tctatggctg tcagtccttt acacgatatg gacgaaaaaa aagataaaga     960
   tacatggaat agtagtgatg ttatacgaaa tggaaagcac tataaaaaac cacactatca    1020
   cgttatatat attgcacgaa atcctgtaac aatagaaagc gttaggaaca agattaagcg    1080
   aaaattgggg aatagttcag ttgctcatgt tgagatactt gattatatca aaggttcgta    1140
   tgaatatttg actcatgaat caaaggacgc tattgctaag aataaacata tatacgacaa    1200
   aaaagatatt ttgaacatta atgattttga tattgaccgc tatataacac ttgatgaaag    1260
   ccaaaaaaga gaattgaaga atttactttt agatatagtg gatgactata atttggtaaa    1320
   tacaaaagat ttaatggctt ttattcgcct taggggagcg gagtttggaa ttttaaatac    1380
   gaatgatgta aaagatattg tttcaacaaa ctctagcgcc tttagattat ggtttgaggg    1440
   caattatcag tgtggatata gagcaagtta tgcaaaggtt cttgatgctg aaacggggga    1500
   aataaaatga c                                                          1511
<210> 3
   <211> 39
   <212> DNA
   <213> Artificial Sequence
<220>
   <223> Terminator sequence 1
<400> 3
   aaaaaaaaag gcctgcgatt acccgcaggc ctttttttt 39
<210> 4
   <211> 47
   <212> DNA
   <213> Artificial Sequence
<220>
   <223> Terminator sequence 2
<400> 4
   tagcataacc ccttggggcc tctaaacggg tcttgagggg ttttttg 47
<210> 5
   <211> 28
   <212> DNA
   <213> Artificial Sequence
<220>
   <223> Terminator sequence 3
<400> 5
   aaaaaaaagc ccgctcatta ggcgggct 28
<210> 6
   <211> 30
   <212> DNA
   <213> Artificial Sequence
<220>
   <223> Terminator sequence 4
<400> 6
   acagaaaagc ccgcctttcg gcgggctttg 30
<210> 7
   <211> 77
   <212> DNA
   <213> Artificial Sequence
<220>
   <223> Promotor sequence
<400> 7
   tgttgacaat taatcatcgg ctcgtataat gtgtggaatt gtgagcggat aacaatttca 60
   cacaggaaac agaattc 77
<210> 8
   <211> 6420
   <212> DNA
   <213> Artificial Sequence
<220>
   <223> Shuttle vector, whole sequence
<400> 8
   cctgtgcaag gagtacttcg taaatcactg cataattcgt gtcgctcaag gcgcactccc     60
   gttctggata atgttttttg cgccgacatc ataacggttc tggcaaatat tctgaaatga    120
   gctgttgaca attaatcatc ggctcgtata atgtgtggaa ttgtgagcgg ataacaattt    180
   cacacaggaa acagaattcc atatggatat cgagctcggt acccggggat cctctagagt    240
   cgacctgcag gcatgcaagc ttctcgagaa aaaaaagccc gctcattagg cgggctaaaa    300
   aaaaagcccg cctaatgagc gggctttttt ttacagaaaa gcccgccttt cggcgggctt    360
   tgagcggccg cgtcatttta tttcccccgt ttcagcatca agaacctttg cataacttgc    420
   tctatatcca cactgataat tgccctcaaa ccataatcta aaggcgctag agtttgttga    480
   aacaatatct tttacatcat tcgtatttaa aattccaaac tccgctcccc taaggcgaat    540
   aaaagccatt aaatcttttg tatttaccaa attatagtca tccactatat ctaaaagtaa    600
   attcttcaat tctctttttt ggctttcatc aagtgttata tagcggtcaa tatcaaaatc 660
   attaatgttc aaaatatctt ttttgtcgta tatatgttta ttcttagcaa tagcgtcctt 720
   tgattcatga gtcaaatatt catacgaacc tttgatataa tcaagtatct caacatgagc 780
   aactgaacta ttccccaatt ttcgcttaat cttgttccta acgctttcta ttgttacagg 840
   atttcgtgca atatatataa cgtgatagtg tggtttttta tagtgctttc catttcgtat 900
   aacatcacta ctattccatg tatctttatc ttttttttcg tccatatcgt gtaaaggact 960
   gacagccata gatacgccca aactctctaa tttttctttc caatcattag gaattgagtc 1020
   aggatataat aaaaatccaa aatttctagc tttagtattt ttaatagcca tgatataatt 1080
   accttatcaa aaacaagtag cgaaaactcg tatccttcta aaaacgcgag ctttcgctta 1140
   ttttttttgt tctgattcct ttcttgcata ttcttctata gctaacgccg caaccgcaga 1200
   ttttgaaaaa cctttttgtt tcgccatatc tgttaatttt ttatcttgct cttttgtcag 1260
   agaaatcata actctttttt tcgattctga aatcaccatt taaaaaactc caatcaaata 1320
   attttataaa gttagtgtat cactttgtaa tcataaaaac aacaataaag ctacttaaat 1380
   atagatttat aaaaaacgtt ggcgaaaacg ttggcgattc gttggcgatt gaaaaacccc 1440
   ttaaaccctt gagccagttg ggatagagcg tttttggcac aaaaattggc actcggcact 1500
   taatgggggg tcgtagtacg gaagcaaaat tcgcttcctt tccccccatt tttttccaaa 1560
   ttccaaattt ttttcaaaaa ttttccagcg ctaccgctcg gcaaaatgca agcaattttt 1620
   aaaatcaaac ccatgaggga atttcattcc ctcatactcc cttgagcctc ctccaaccga 1680
   aatagaaggg cgctgcgctt attatttcat tcagtcatcg gctttcataa tctaacagac 1740
   aacatcttcg ctgcaaagcc acgctacgct caagggcttt tacgctacga taacgcctgt 1800
   tttaacgatt atgccgataa ctaaacgaaa taaacgctaa aacgtctcag aaacgatttt 1860
   gagacgtttt aataaaaaat cggcggccgc aaaaaaaaag gcctgcgatt acccgcaggc 1920
   cttttttttg ctcttcgcga ctagttttac aaaagccagt cattaggcct atctgacaat 1980
   tcctgaatag agttcataaa caatcctgca tgataaccat cacaaacaga atgatgtacc 2040
   tgtaaagata gcggtaaata tattgaatta cctttattaa tgaattttcc tgctgtaata 2100
   atgggtagaa ggtaattact attattattg atatttaagt taaacccagt aaatgaagtc 2160
   catggaataa tagaaagaga aaaagcattt tcaggtatag gtgttttggg aaacaatttc 2220
   cccgaaccat tatatttctc tacatcagaa aggtataaat cataaaactc tttgaagtca 2280
   ttctttacag gagtccaaat accagagaat gttttagata caccatcaaa aattgtataa 2340
   agtggctcta acttatccca ataacctaac tctccgtcgc tattgtaacc agttctaaaa 2400
   gctgtatttg agtttatcac ccttgtcact aagaaaataa atgcagggta aaatttatat 2460
   ccttcttgtt ttatgtttcg gtataaaaca ctaatatcaa tttctgtggt tatactaaaa 2520
   gtcgtttgtt ggttcaaata atgattaaat atctcttttc tcttccaatt gtctaaatca 2580
   attttattaa agttcatttg atatgcctcc taaattttta tctaaagtga atttaggagg 2640
   cttacttgtc tgctttcttc attagaatca atcccacgtg agatcttttt ttaaaagtca 2700
   atattactgt aacataaata tatattttaa aaatatccca ctttatccaa ttttcgtttg 2760
   ttgaactaat gggtgcttta gttgaagatg cttgagactg gccgccacgt tgcccatttt 2820
   cgccagcttc ttgcatcgca tgatcgcgta tgccgccatg cctgcccctc ccttttggtg 2880
   tccaaccggc tcgacggggg cagcgcaagg cggtgcctcc ggcgggccac tcaatgcttg 2940
   agtatactca ctagactttg cttcgcaaag tcgtgaccgc ctacggcggc tgcggcgccc 3000
   tacgggcttg ctctccgggc ttcgccctgc gcggtcgctg cgctcccttg ccagcccgtg 3060
   gatatgtgga cgatggccgc gagcggccac cggctggctc gcttcgctcg gcccgtggac 3120
   aaccctgctg gacaagctga tggacaggct gcgcctgccc acgagcttga ccacagggat 3180
   tgcccaccgg ctacccagcc ttcgaccaca tacccaccgg ctccaactgc gcggcctgcg 3240
   gccttgcccc atcaattttt ttaattttct ctggggaaaa gcctccggcc tgcggcctgc 3300
   gcgcttcgct tgccggttgg acaccaagtg gaaggcgggt caaggctcgc gcagcgaccg 3360
   cgcagcggct tggccttgac gcgcctggaa cgacccaagc ctatgcgagt gggggcagtc 3420
   gaaggcgaag cccgcccgcc tgccccccga gcctcacggc ggcgagtgcg ggggttccaa 3480
   gggggcagcg ccaccttggg caaggccgaa ggccgcgcag tcgatcaaca agccccggag 3540
   gggccacttt ttgccggagg gggagccgcg ccgaaggcgt gggggaaccc cgcaggggtg 3600
   cccttctttg ggcaccaaag aactagatat agggcgaaat gcgaaagact taaaaatcaa 3660
   caacttaaaa aaggggggta cgcaacagct cattgcggca ccccccgcaa tagctcattg 3720
   cgtaggttaa agaaaatctg taattgactg ccacttttac gcaacgcata attgttgtcg 3780
   cgctgccgaa aagttgcagc tgattgcgca tggtgccgca accgtgcggc accctaccgc 3840
   atggagataa gcatggccac gcagtccaga gaaatcggca ttcaagccaa gaacaagccc 3900
   ggtcactggg tgcaaacgga acgcaaagcg catgaggcgt gggccgggct tattgcgagg 3960
   aaacccacgg cggcaatgct gctgcatcac ctcgtggcgc agatgggcca ccagaacgcc 4020
   gtggtggtca gccagaagac actttccaag ctcatcggac gttctttgcg gacggtccaa 4080
   tacgcagtca aggacttggt ggccgagcgc tggatctccg tcgtgaagct caacggcccc 4140
   ggcaccgtgt cggcctacgt ggtcaatgac cgcgtggcgt ggggccagcc ccgcgaccag 4200
   ttgcgcctgt cggtgttcag tgccgccgtg gtggttgatc acgacgacca ggacgaatcg 4260
   ctgttggggc atggcgacct gcgccgcatc ccgaccctgt atccgggcga gcagcaacta 4320
   ccgaccggcc ccggcgagga gccgcccagc cagcccggca ttccgggcat ggaaccagac 4380
   ctgccagcct tgaccgaaac ggaggaatgg gaacggcgcg ggcagcagcg cctgccgatg 4440
   cccgatgagc cgtgttttct ggacgatggc gagccgttgg agccgccgac acgggtcacg 4500
   ctgccgcgcc ggtagcactt gggttgcgca gcaacccgta agtgcgctgt tccagactat 4560
   cggctgtagc cgcctcgccg ccctatacct tgtctgcctc cccgcgttgc gtcgcggtgc 4620
   atggagccgg gccacctcga cctgaatgga agccggcggc acctcgctaa cggattcacc 4680
   gtttttatca ggctctggga ggcagaataa atgatcatat cgtcaattat tacctccacg 4740
   gggagagcct gagcaaactg gcctcaggca tttgagaagc acacggtcac actgcttccg 4800
   gtagtcaata aaccggtaaa ccagcaatag acataagcgg ctatttaacg accctgccct 4860
   gaaccgacga ccgggtcgaa tttgctttcg aatttctgcc attcatccgc ttattatcac 4920
   ttattcaggc gtagcaccag gcgtttaagg gcaccaataa ctgccttaaa aaaattacgc 4980
   cccgccctgc cactcatcgc agtcggccta ttggttaaaa aatgagctga tttaacaaaa 5040
   atttaacgcg aattttaaca aaatattaac gcttacaatt tccattcgcc attcaggctg 5100
   cgcaactgtt gggaagggcg atagatctta gcataacccc ttggggcctc taaacgggtc 5160
   ttgaggggtt ttttgccact gcccgctttc cagtcgggaa acctgtcgtg ccagctgcat 5220
   taatgaatcg gccaacgcgc ggggagaggc ggtttgcgta ttgggcgcca gggtggtttt 5280
   tcttttcacc agtgagacgg gcaacagctg attgcccttc accgcctggc cctgagagag 5340
   ttgcagcaag cggtccacgc tggtttgccc cagcaggcga aaatcctgtt tgatggtggt 5400
   tgacggcggg atataacatg agctgtcttc ggtatcgtcg tatcccacta ccgagatgtc 5460
   cgcaccaacg cgcagcccgg actcggtaat ggcgcgcatt gcgcccagcg ccatctgatc 5520
   gttggcaacc agcatcgcag tgggaacgat gccctcattc agcatttgca tggtttgttg 5580
   aaaaccggac atggcactcc agtcgccttc ccgttccgct atcggctgaa tttgattgcg 5640
   agtgagatat ttatgccagc cagccagacg cagacgcgcc gagacagaac ttaatgggcc 5700
   cgctaacagc gcgatttgct ggtgacccaa tgcgaccaga tgctccacgc ccagtcgcgt 5760
   accgtcttca tgggagaaaa taatactgtt gatgggtgtc tggtcagaga catcaagaaa 5820
   taacgccgga acattagtgc aggcagcttc cacagcaatg gcatcctggt catccagcgg 5880
   atagttaatg atcagcccac tgacgcgttg cgcgagaaga ttgtgcaccg ccgctttaca 5940
   ggcttcgacg ccgcttcgtt ctaccatcga caccaccacg ctggcaccca gttgatcggc 6000
   gcgagattta atcgccgcga caatttgcga cggcgcgtgc agggccagac tggaggtggc 6060
   aacgccaatc agcaacgact gtttgcccgc cagttgttgt gccacgcggt tgggaatgta 6120
   attcagctcc gccatcgccg cttccacttt ttcccgcgtt ttcgcagaaa cgtggctggc 6180
   ctggttcacc acgcgggaaa cggtctgata agagacaccg gcatactctg cgacatcgta 6240
   taacgttact ggtttcacat tcaccaccct gaattgactc tcttccgggc gctatcatgc 6300
   cataccgcga aaggttttgc accattcgat ggtgtcaacg taaatgccgc ttcgccttcg 6360
   gcgcgcgaat tgcaagctga tccggcggag cttatcgact gcacggtggc tgtggtatgg 6420
<210> 9
   <211> 751
   <212> DNA
   <213> Artificial Sequence
<220>
   <223> Chloramphenicol acetyltransferase cassette (PmlI to SapI)
<400> 9
   cacgtgggat tgattctaat gaagaaagca gacaagtaag cctcctaaat tcactttaga 60
   taaaaattta ggaggcatat caaatgaact ttaataaaat tgatttagac aattggaaga 120
   gaaaagagat atttaatcat tatttgaacc aacaaacgac ttttagtata accacagaaa 180
   ttgatattag tgttttatac cgaaacataa aacaagaagg atataaattt taccctgcat 240
   ttattttctt agtgacaagg gtgataaact caaatacagc ttttagaact ggttacaata 300
   gcgacggaga gttaggttat tgggataagt tagagccact ttatacaatt tttgatggtg 360
   tatctaaaac attctctggt atttggactc ctgtaaagaa tgacttcaaa gagttttatg 420
   atttatacct ttctgatgta gagaaatata atggttcggg gaaattgttt cccaaaacac 480
   ctatacctga aaatgctttt tctctttcta ttattccatg gacttcattt actgggttta 540
   acttaaatat caataataat agtaattacc ttctacccat tattacagca ggaaaattca 600
   ttaataaagg taattcaata tatttaccgc tatctttaca ggtacatcat tctgtttgtg 660
   atggttatca tgcaggattg tttatgaact ctattcagga attgtcagat aggcctaatg 720
   actggctttt gtaaaactag tcgcgaagag c 751
<210> 10
   <211> 1255
   <212> DNA
   <213> Artificial Sequence
<220>
   <223> lacI cassette (EcoNI to Stop)
<400> 10
   ccttgcacag gccataccac agccaccgtg cagtcgataa gctccgccgg atcagcttgc 60
   aattcgcgcg ccgaaggcga agcggcattt acgttgacac catcgaatgg tgcaaaacct 120
   ttcgcggtat ggcatgatag cgcccggaag agagtcaatt cagggtggtg aatgtgaaac 180
   cagtaacgtt atacgatgtc gcagagtatg ccggtgtctc ttatcagacc gtttcccgcg 240
   tggtgaacca ggccagccac gtttctgcga aaacgcggga aaaagtggaa gcggcgatgg 300
   cggagctgaa ttacattccc aaccgcgtgg cacaacaact ggcgggcaaa cagtcgttgc 360
   tgattggcgt tgccacctcc agtctggccc tgcacgcgcc gtcgcaaatt gtcgcggcga 420
   ttaaatctcg cgccgatcaa ctgggtgcca gcgtggtggt gtcgatggta gaacgaagcg 480
   gcgtcgaagc ctgtaaagcg gcggtgcaca atcttctcgc gcaacgcgtc agtgggctga 540
   tcattaacta tccgctggat gaccaggatg ccattgctgt ggaagctgcc tgcactaatg 600
   ttccggcgtt atttcttgat gtctctgacc agacacccat caacagtatt attttctccc 660
   atgaagacgg tacgcgactg ggcgtggagc atctggtcgc attgggtcac cagcaaatcg 720
   cgctgttagc gggcccatta agttctgtct cggcgcgtct gcgtctggct ggctggcata 780
   aatatctcac tcgcaatcaa attcagccga tagcggaacg ggaaggcgac tggagtgcca 840
   tgtccggttt tcaacaaacc atgcaaatgc tgaatgaggg catcgttccc actgcgatgc 900
   tggttgccaa cgatcagatg gcgctgggcg caatgcgcgc cattaccgag tccgggctgc 960
   gcgttggtgc ggacatctcg gtagtgggat acgacgatac cgaagacagc tcatgttata 1020
   tcccgccgtc aaccaccatc aaacaggatt ttcgcctgct ggggcaaacc agcgtggacc 1080
   gcttgctgca actctctcag ggccaggcgg tgaagggcaa tcagctgttg cccgtctcac 1140
   tggtgaaaag aaaaaccacc ctggcgccca atacgcaaac cgcctctccc cgcgcgttgg 1200
   ccgattcatt aatgcagctg gcacgacagg tttcccgact ggaaagcggg cagtg 1255
<210> 11
   <211> 22
   <212> DNA
   <213> Artificial Sequence
<220>
   <223> lac operator cassette
<400> 11
   gaattgtgag cggataacaa tt 22
<210> 12
   <211> 19
   <212> DNA
   <213> Artificial Sequence
<220>
   <223> Primer sequence pPR-HisT_1
<400> 12
   caccgtgcag tcgataagc 19
<210> 13
   <211> 18
   <212> DNA
   <213> Artificial Sequence
<220>
   <223> Primer sequence pPR-HisT_2
<400> 13
   gctgtggtat ggcctgtg 18
<210> 14
   <211> 23
   <212> DNA
   <213> Artificial Sequence
<220>
   <223> Primer sequence GA_pPR_1
<400> 14
   cacattcacc accctgaatt gac 23
<210> 15
   <211> 26
   <212> DNA
   <213> Artificial Sequence
<220>
   <223> Primer sequence GA_pPR_2
<400> 15
   gatctcacgt gggattgatt ctaatg 26
<210> 16
   <211> 60
   <212> DNA
   <213> Artificial Sequence
<220>
   <223> Primer sequence GA_pPR_3
<400> 16
   tagcataacc ccttggggcc tctaaacggg tcttgagggg ttttttgtca ctgcccgctt 60
<210> 17
   <211> 23
   <212> DNA
   <213> Artificial Sequence
<220>
   <223> Primer sequence GA_pPR_4
<400> 17
   gtcaattcag ggtggtgaat gtg 23
<210> 18
   <211> 26
   <212> DNA
   <213> Artificial Sequence
<220>
   <223> Primer sequence GA_pPR_5
<400> 18
   cattagaatc aatcccacgt gagatc 26
<210> 19
   <211> 60
   <212> DNA
   <213> Artificial Sequence
<220>
   <223> Primer sequence GA_pPR_6
<400> 19
   caaaaaaccc ctcaagaccc gtttagaggc cccaaggggt tatgctaaga tctatcgccc 60
<210> 20
   <211> 55
   <212> DNA
   <213> Artificial Sequence
<220>
   <223> Primer sequence pWV01_opt_1
<400> 20
   aaggaaaaaa gcggccgccg attttttatt aaaacgtctc aaaatcgttt ctgag 55
<210> 21
   <211> 46
   <212> DNA
   <213> Artificial Sequence
<220>
   <223> Primer sequence pWV01_opt_2
<400> 21
   aaggaaaaaa gcggccgcgt cattttattt cccccgtttc agcatc 46
<210> 22
   <211> 24
   <212> DNA
   <213> Artificial Sequence
<220>
   <223> Primer sequence GFP_for
<400> 22
   atggtccaaa ctagttcgaa gatc 24
<210> 23
   <211> 49
   <212> DNA
   <213> Artificial Sequence
<220>
   <223> Primer sequence GFP_His6_rev
<400> 23
   gctctagact aatgatgatg gtgatgatgt ttgtagggct catccatgc 49